# EUROPEAN PATENT APPLICATION

(11) **EP 0 609 701 A1**
(43) Date of publication of application: **10.08.1994**
(21) Application number: 94100732.0
(22) Date of filing: 19.01.1994
(51) Int. Cl.: A61K 31/195

(54) **L-cystein, L-cystine or L-glutamine as a supplementary therapeutic agent for the treatment of immunodeficiency syndrome**

(30) Priority: 27.01.1993 JP 29668/93; 27.12.1993 JP 346875/93
(71) Applicant: Ajinomoto Co., Inc., Tokyo 104 (JP)
(72) Inventor: Aoki, Tadao, Nigata-shi, Nigata-ken (JP); Miyakoshi, Hideo, Hachiohji-shi, Tokyo (JP)
(74) Representative: Strehl Schübel-Hopf Groening & Partner

(57) **Abstract**

A supplementary therapeutic agent for the treatment of immunodeficiency syndrome with reduced NK activity and an immunostimulant preparation containing it are provided.

A supplementary therapeutic agent for immunostimulation which comprises L-cysteine, L-cystine or L-glutamine or a salt thereof and is used in combination with an immunostimulant such as lentinan, OK-432, sizofiran or the like, and an immunostimulant preparation containing the agent.

## Description

### Field of the Invention

The present invention relates to a supplementary therapeutic agent which is employed when satisfactory effects are not produced by the use of an immunostimulant, or a therapeutic agent for treating an immunodeficiency syndrome, such as chronic fatigue syndrome, low NK syndrome, acquired immunodeficiency syndrome or congenital immunodeficiency syndrome.

### Description of the Prior Art

The therapeutic agents of the prior art for the treatment of an immunodeficiency syndrome, such as chronic fatigue syndrome, low NK (natural killer) syndrome, acquired immunodeficiency syndrome or congenital immunodeficiency syndrome include immunostimulants such as lentinan, OK-432, sizofiran, etc. (New Journal of Japanese Medicine, No. 3212, 11/16/'85).

In patients suffering from such syndromes for several years, however, often the expected therapeutic effects are not produced with administration thereto of such immmunostimulants alone.

### Subject matter of the Invention

The present inventors have analyzed the amino acid concentration in the blood of patients suffering from the syndromes for several years, who, however, did not benefit from the therapeutic effects expected by the administration of immunostimulants. As a result, it was found that the concentrations of L-glutamine and L-cystine are lower in the blood of those patients than in that of healthy persons.

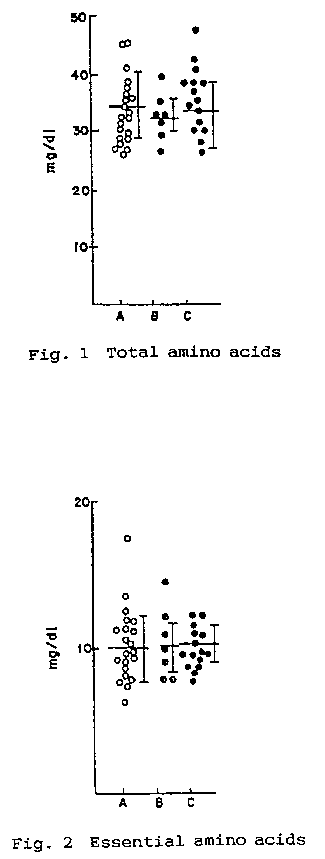

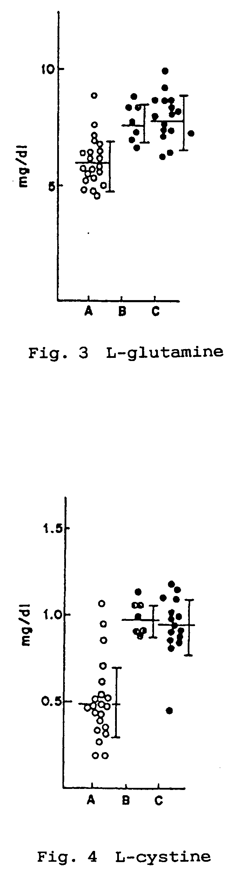

Figs. 1-4 show the results of the analysis of the levels of amino acids in the blood. Fig. 1-4 show the total amino acids, essential amino acids, L-glutamine and L-cystine, respectively.

In the respective drawings, the concentration of each of the amino acids is plotted along the vertical axis, while A, B and C on the horizontal axis show the concentration of amino acids in the blood of patients suffering from immunodeficiency syndrome, that in the blood of patients with malaise and febricula, but without immunodeficiency, and that in the blood of healthy persons, respectively.

The results shown in Figs. 1-4 have revealed that the patients suffering from an immunodeficiency syndrome have a lowered concentration of L-glutamine and L-cystine in the blood, particularly the concentration of L-cystine in the blood thereof being lowered to almost half that of the healthy persons.

Therefore, L-glutamine or L-cystine, particularly L-cystine, was administered to patients who were receiving immunostimulants, and it has been found that the clinical condition of the patients can be improved.

Accordingly, the present invention is directed to provide L-cysteine, L-cystine or L-glutamine or a salt thereof as a supplementary therapeutic agent for the treatment of immunodeficiency syndrome, and a therapeutic agent for the treatment of immunodeficiency syndrome which contains L-cysteine, L-cystine or L-glutamine or a salt thereof and an immunostimulant as effective ingredients.

### Detailed Description of the Invention

The present invention is concerned with
1) a supplementary therapeutic agent for the treatment of an immunodeficiency syndrome which contains L-cysteine, L-cystine or L-glutamine or a salt thereof as an effective ingredient; and
2) a therapeutic agent for the treatment of an immunodeficiency syndrome which contains L-cysteine, L-cystine or L-glutamine or a salt thereof and an immunostimulant as effective ingredients.

On alternate days L-cystine was administered to patients receiving immunostimulants, who could not have experienced an improvement of clinical condition upon the administration of therapeutic agents (immunostimulants) alone for the treatment of an immunodeficiency syndrome with reduced NK activity, particularly those patients suffering from said syndromes for 5 or more years. It has been found in many cases that its administration over a period of several months contributes to the improvement of the clinical condition and to the normalization of immunity. The supplementary therapeutic agent according to the present invention may be presented in various forms such as dusting powders, powders, granules, tablets, sugar-coated tablets, capsules, solutions, etc. as preparations for oral administration, and L-cysteine, L-cystine or L-glutamine or a salt thereof comprises 0.01-100% by weight of the preparations.

The parenteral preparations thereof which may be presented include suspensions, solutions, emulsions, ampoules and injections, where the contents of the effective ingredients may be determined arbitrarily. The dose of the supplementary therapeutic agent is 1-500 mg per day for adults in terms of the effective ingredients.

L-Cysteine, L-cystine and L-glutamine or the effective ingredients of the supplementary therapeutic agent according to the present invention are amino acids which are contained in native proteins, and thus no toxicity problem occurs from their use in amounts within the range specified by the present invention. Usually, the therapeutic agent according to the present invention, which comprises L-cysteine, L-cystine or L-glutamine or a salt thereof and a therapeutic agent (immunostimulant) for the treatment of immunodeficiency syndrome, is presented as an injection.

The dose for adults is 1-500 mg per day in terms of the amino acids referred to above, and 1-20 mg per week in terms of the immunostimulants, and they are mixed under these conditions for administration.

The immunostimulant usually used includes lentinan, OK-432 or sizofiran which are now used as antimalignant drugs.

Hereunder, detailed explanation will be made of the present invention with reference to the Example, to which, however, the present invention is in no way limited.

### Example 1

A 58-year-old male patient suffering from chronic fatigue syndrome with low NK activity, accompanied by (1) fever, (2) excessive systemic malaise or dullness and (3) tinnitus over 10 or more years, had an NK activity of 18.7% (normal value: 33% or higher) when he was hospitalized 6 years after the onset of the illness. To this patient was administered 1 mg of immunostimulant lentinan by intravenous drip infusion every other day for half a year or more, but the condition was alternately improving and deteriorating, with only a slight improvement of the symptoms (1) and (2) mentioned above. Also, the NK activity showed a slight increase, but was not restored to the normal value.

Thus, in view of the L-cystine level in the peripheral blood of the patient, which had been reduced to half of the normal value, 200 mg of L-cystine were given by intravenous drip infusion every other day to the patient who was receiving lentinan as before, and 1 month thereafter the drip infusion was replaced by the oral administration of a capsule containing 400 mg of L-cystine which was given on alternate days for 1 month. This resulted in a gradual improvement of the clinical condition and also an increase in the NK activity to values of 22%, 24.9%, 40.9% and 39% which were observed every week beginning 1 month after the initiation of the combined use of L-cystine with lentinan. Suspension of the administrations of L-cystine and also of the immunostimulant 2 weeks thereafter, however, led to a decrease in the NK activity to 23.3% 3 weeks later, and to a relapse into the aggravated clinical condition.

On the basis of the aforementioned results, it is expected that chronic fatigue syndrome with low NK activity may be cured by the administration of both the above amino acid and decreased doses, but not with complete discontinuation of the administration of both. administration of both.

The foregoing results show that the administration of an immunostimulant in combination with L-cystine promoted the NK activity. With the administration of L-cystine alone, however, no improvement of the clinical condition or increase in the NK activity was observed.

Cultivation of human lymphocytes in test tubes with addition of L-cystine or L-glutamine thereto caused activation of the lymphocytes, which suggests that activation of NK cells in the body of the patient was promoted with L-cystine or L-glutamine. Here, L-cysteine is easily oxidized and changed to L-cystine in the blood, and thus acts in the same manner as L-cystine.

### Effects of the Invention

L-Cysteine, L-cystine or L-glutamine increases NK activity when used in combination with an immunostimulant, and thus is useful as a supplementary therapeutic agent for the treatment of an immunodeficiency, such as low NK activity syndrome, chronic fatigue syndrome, acquired immunodeficiency syndrome, or congenital immunodeficiency syndrome etc.

## Claims

1. The use of L-cysteine, L-cystine or L-glutamine or a salt thereof as a supplementary therapeutic agent for the production of a therapeutic composition for the treatment of an immunodeficiency syndrome.

2. A therapeutic composition for the treatment of an immunodeficiency syndrome which contains L-cysteine, L-cystine or L-glutamine or a salt thereof and an immunostimulant as effective ingredients.
